# EUROPÄISCHE PATENTSCHRIFT

(11) **EP 0 764 625 B1**
(45) Veröffentlichungstag und Bekanntmachung des Hinweises auf die Patenterteilung: **07.04.1999**
(21) Anmeldenummer: 96114803.8
(22) Anmeldetag: 16.09.1996
(51) Int. Cl.: C07C 45/86, C07C 49/17, C07C 49/175

(54) **Verfahren zur Verbesserung der Lagerfähigkeit flüssiger Hydroxy- und Alkoxyalkylketone**
Process for improving the storability of liquid hydroxy- and alkoxyalkylketones
Procédé d'amélioration de l'aptitude au stockage d'hydroxy- et alkoxyalkylcétones liquides

(30) Priorität: 23.09.1995 DE 19535404
(43) Veröffentlichungstag der Anmeldung: 26.03.1997
(73) Patentinhaber: BASF AKTIENGESELLSCHAFT, 67056 Ludwigshafen (DE)
(72) Erfinder: Ross, Karl-Heinz, Dr., 67269 Grünstadt (DE); Wambach, Ludwig, Dr., 68723 Schwetzingen (DE); Wache, Harro, Dr., 67136 Fussgönheim (DE)

(56) Entgegenhaltungen:
- US-A- 2 444 006
- CHEMICAL ABSTRACTS, vol. 088, no. 11, 13.März 1978 Columbus, Ohio, US; abstract no. 074049, MIYAMOTO T ET AL: "4-Methoxy-4-methylpentan-2-one purification" XP002019451 & JP 52 116 407 - (MITSUBISHI CHEMICAL INDUSTRIES CO., LTD.;JAPAN) 29.September 1977

## Beschreibung

Die vorliegende Erfindung betrifft ein Verfahren zur Verbesserung der Lagerfähigkeit flüssiger Hydroxy- und Alkoxyalkylketone durch Zusatz kleiner Mengen einer Base.

Flüssige Hydroxy- und Alkoxyalkylketone, wie z.B. Hydroxyaceton, neigen bei Lagerung zu Nebenreaktionen. Dadurch nimmt der Gehalt der gelagerten Ware ab, wird dadurch verunreinigt und z.T. verfärbt.

Der vorliegenden Erfindung lag daher die Aufgabe zugrunde, den zuvor genannten Nachteilen abzuhelfen.

Demgemäß wurde ein neues Verfahren zur Verbesserung der Lagerfähigkeit flüssiger Hydroxy- und Alkoxyalkylketone gefunden, welches dadurch gekennzeichnet ist, daß man 0,0001 bis 1 Gew.-% einer Base zusetzt.

Als Hydroxyalkylketon eignen sich Hydroxyaceton, 1-Hydroxybutan-2-on oder deren Gemische, bevorzugt Hydroxyaceton.

Als Alkoxyalkylketon eignen sich Methoxy-2-propanon, Ethoxy-2-propanon, n-Propoxy-2-propanon, iso-Propoxy-2-propanon, n-Butoxy-2-propanon, iso-Butoxy-2-propanon, sec.-Butoxy-2-propanon, tert.-Butoxy-2-propanon, Methoxy-2-butanon, Ethoxy-2-butanon, n-Propoxy-2-butanon, iso-Propoxy-2-butanon, n-Butoxy-2-butanon, iso-Butoxy-2-butanon, sec.-Butoxy-2-butanon, tert.-Butoxy-2-butanon, 1-Methoxy-butan-3-on, 1-Ethoxybutan-3-on, 1-Methoxy-1-buten-3-on, Ethoxy-1-buten-3-on oder deren Gemische, bevorzugt Methoxy-2-propanon, Ethoxy-2-propanon, n-Propoxy-2-propanon, Methoxy-2-butanon, Ethoxy-2-butanon, n-Propoxy-2-butanon oder deren Gemische, besonders bevorzugt Methoxy-2-propanon und Methoxy-2-butanon.

Man setzt in der Regel zu den zu stabilisierenden flüssigen Hydroxy- und Alkoxyalkylketonen 0,0001 bis 1 Gew.-%, bevorzugt 0,001 bis 0,1 Gew.-%, besonders bevorzugt 0,005 bis 0,05 Gew.-% einer Base zu.

Als Basen eignen sich tertiäre Amine, bevorzugt tertiäre Alkylamine, besonders bevorzugt Trialkylamine wie Trimethylamin, Dimethylethylamin, Methyldiethylamin, Triethylamin, Tri-n-propylamin, Tri-iso-propylamin oder Tetramethylethandiamin, Tetramethyl-n-propandiamin, Tetramethyl-n-butandiamin, Tetramethyl-n-pentandiamin Tetramethyl-n-hexandiamin oder feste bzw. wäßrige Lösungen von Alkalihydroxiden, Alkalicarbonaten, Alkalihydrogencarbonaten, Erdalkalihydroxiden, Erdalkalicarbonaten und Erdalkalihydrogencarbonaten wie Lithiumhydroxid, Natriumhydroxid, Kaliumhydroxid, Calciumhydroxid, Magnesiumhydroxid, Lithiumcarbonat, Natriumcarbonat, Kaliumcarbonat, Calciumcarbonat, Magnesiumcarbonat, Lithiumhydrogencarbonat, Natriumhydrogencarbonat, Kaliumhydrogencarbonat, Calciumhydroxycarbonat, Magnesiumhydroxycarbonat oder deren Gemische, bevorzugt Natriumhydroxid, Kaliumhydroxid, Natriumcarbonat, Kaliumcarbonat, oder deren Gemische.

### Beispiele

### Vergleichsbeispiel A

200 ml frisch destilliertes Hydroxyaceton wurden bei Raumtemperatur (25°C) aufbewahrt. Die Ergebnisse sind in Tabelle A zusammengefaßt.

**Tabelle A:**

| unstabilisiertes Hydroxyaceton | |
|---|---|
| Wochen | Gehalt [Gew.-%] |
| 0 | 97,8 |
| 3 | 91,8 |
| 7 | 91,3 |

### Beispiel 1

200 ml frisch destilliertes Hydroxyaceton wurde mit N,N,N',N'-Tetramethylhexandiamin (TMHDA) versetzt, so daß der Gehalt 100 ppm betrug. Die Ergebnisse sind in Tabelle 1 zusammengefaßt.

**Tabelle 1:**

| mit 100 ppm N,N,N',N'-Tetramethylhexandiamin stabilisiertes Hydroxyaceton | | |
|---|---|---|
| Wochen | Gehalt [Gew.-%] ohne TMHDA | Gehalt [Gew.-%] mit 100 ppm TMHDA |
| 0 | 97,8 | 97,8 |
| 3 | 91,8 | 97,8 |
| 7 | 91,3 | 97,6 |
| 9 | ---- | 97 |

### Beispiel 2

200 ml frisch destilliertes Hydroxyaceton wurde mit N,N,N',N'-Tetramethylhexandiamin versetzt, so daß der Gehalt 250 ppm betrug. Die Ergebnisse sind in Tabelle 2 zusammengefaßt.

**Tabelle 2:**

| mit 250 ppm N,N,N',N'-Tetramethylhexandiamin stabilisiertes Hydroxyaceton | | |
|---|---|---|
| Wochen | Gehalt [Gew.-%] ohne TMHDA | Gehalt [Gew.-%] mit 250 ppm TMHDA |
| 0 | 97,8 | 97,8 |
| 3 | 91,8 | 97,8 |
| 7 | 91,3 | 97,6 |
| 9 | ---- | 97,6 |

### Beispiele 3 und 4

200 ml frisch destilliertes Hydroxyaceton wurde mit einer 25 %igen wäßrigen Natriumcarbonatlösung versetzt, so daß der Gehalt an Natriumcarbonat 250 ppm betrug. In einem weiteren Versuch wurde der Gehalt auf 10 ppm Natriumcarbonat eingestellt. Die Ergebnisse sind in Tabelle 3 zusammengefaßt.

**Tabelle 3:**

| mit Natriumcarbonat stabilisiertes Hydroxyaceton | | | |
|---|---|---|---|
| Wochen | Gehalt [Gew.-%] ohne Natriumcarbonat | Gehalt [Gew.-%] mit 10 ppm Natriumcarbonat | Gehalt [Gew.-%] mit 250 ppm Natriumcarbonat |
| 0 | 97,8 | 97,8 | 97,8 |
| 3 | 91,8 | 96 | 97,8 |
| 7 | 91,3 | 92,3 | 97,6 |
| 9 | ---- | ---- | 97,6 |

## Patentansprüche

1. Verfahren zur Verbesserung der Lagerfähigkeit flüssiger Hydroxy- und Alkoxyalkylketone, dadurch gekennzeichnet, daß man 0,0001 bis 1 Gew.-% einer Base zusetzt.

2. Verfahren zur Verbesserung der Lagerfähigkeit flüssiger Hydroxy- und Alkoxyalkylketone nach Anspruch 1, dadurch gekennzeichnet, daß man als Hydroxyalkylketon Hydroxyaceton, 1-Hydroxybutan-2-on oder deren Gemische einsetzt.

3. Verfahren zur Verbesserung der Lagerfähigkeit flüssiger Hydroxy- und Alkoxyalkylketone nach Anspruch 1, dadurch gekennzeichnet, daß man als Alkoxyalkylketon Methoxy-2-propanon, Ethoxy-2-propanon, n-Propoxy-2-propanon, iso-Propoxy-2-propanon, n-Butoxy-2-propanon, iso-Butoxy-2-propanon, sec.-Butoxy-2-propanon, tert.-Butoxy-2-propanon, Methoxy-2-butanon, Ethoxy-2-butanon, n-Propoxy-2-butanon, iso-Propoxy-2-butanon, n-Butoxy-2-butanon, iso-Butoxy-2-butanon, sec.-Butoxy-2-butanon, tert.-Butoxy-2-butanon oder deren Gemische einsetzt.

4. Verfahren zur Verbesserung der Lagerfähigkeit flüssiger Hydroxy- und Alkoxyalkylketone nach den Ansprüchen 1 bis 3, dadurch gekennzeichnet, daß man 0,001 bis 0,1 Gew-% einer Base zusetzt.

5. Verfahren zur Verbesserung der Lagerfähigkeit flüssiger Hydroxy- und Alkoxyalkylketone nach den Ansprüchen 1 bis 4, dadurch gekennzeichnet, daß man 0,005 bis 0,05 Gew.-% einer Base zusetzt.

6. Verfahren zur Verbesserung der Lagerfähigkeit flüssiger Hydroxy- und Alkoxyalkylketone nach den Ansprüchen 1 bis 5, dadurch gekennzeichnet, daß man als Base tertiäre Amine, Alkalicarbonate, Alkalihydroxide oder deren Gemische einsetzt.

7. Verfahren zur Verbesserung der Lagerfähigkeit flüssiger Hydroxy- und Alkoxyalkylketone nach den Ansprüchen 1 bis 6, dadurch gekennzeichnet, daß man als Base Tripropylamin, Tributylamin, Tetramethylhexandiamin oder deren Gemische einsetzt.

8. Verfahren zur Verbesserung der Lagerfähigkeit flüssiger Hydroxy- und Alkoxyalkylketone nach den Ansprüchen 1 bis 7, dadurch gekennzeichnet, daß man als Base Natriumcarbonat, Natriumhydroxid, Kaliumcarbonat, Kaliumhydroxid oder deren Gemische in fester Form oder als wäßrige Lösung einsetzt.

## Claims

1. A method for increasing the shelf life of liquid hydroxy- and alkoxyalkyl ketones, which comprises adding from 0.0001 to 1% by weight of a base.

2. A method for increasing the shelf life of liquid hydroxy- and alkoxyalkyl ketones as claimed in claim 1, wherein the hydroxyalkyl ketone employed is hydroxyacetone, 1-hydroxybutan-2-one or mixtures thereof.

3. A method for increasing the shelf life of liquid hydroxy- and alkoxyalkyl ketones as claimed in claim 1, wherein the alkoxyalkyl ketone employed is methoxy-2-propanone, ethoxy-2-propanone, n-propoxy-2-propanone, isopropoxy-2-propanone, n-butoxy-2-propanone, isobutoxy-2-propanone, sec-butoxy-2-propanone, tert-butoxy-2-propanone, methoxy-2-butanone, ethoxy-2-butanone, n-propoxy-2-butanone, isopropoxy-2-butanone, n-butoxy-2-butanone, isobutoxy-2-butanone, sec-butoxy-2-butanone, tert-butoxy-2-butanone or mixtures thereof.

4. A method for increasing the shelf life of liquid hydroxy- and alkoxyalkyl ketones as claimed in claims 1 to 3, wherein from 0.001 to 0.1% by weight of a base is added.

5. A method for increasing the shelf life of liquid hydroxy- and alkoxyalkyl ketones as claimed in claims 1 to 4, wherein from 0.005 to 0.05% by weight of a base is added.

6. A method for increasing the shelf life of liquid hydroxy- and alkoxyalkyl ketones as claimed in claims 1 to 5, wherein the bases employed are tertiary amines, alkali metal carbonates, alkali metal hydroxides or mixtures thereof.

7. A method for increasing the shelf life of liquid hydroxy- and alkoxyalkyl ketones as claimed in claims 1 to 6, wherein the base employed is tripropylamine, tributylamine, tetramethylhexanediamine or mixtures thereof.

8. A method for increasing the shelf life of liquid hydroxy- and alkoxyalkyl ketones as claimed in claims 1 to 7, wherein the base employed is sodium carbonate, sodium hydroxide, potassium carbonate, potassium hydroxide or mixtures thereof in solid form or as an aqueous solution.

## Revendications

1. Procédé d'amélioration de la tenue au stockage d'hydroxy- et d'alcoxyalkylcétones liquides, caractérisé en ce qu'on leur ajoute 0,0001 à 1% en poids d'une base.

2. Procédé d'amélioration de la tenue au stockage d'hydroxy- et d'alcoxyalkylcétones liquides selon la revendication 1, caractérisé en ce que l'on utilise de l'hydroxyacétone, de la 1-hydroxybutan-2-one ou leurs mélanges, en tant qu'hydroxyalkylcétone.

3. Procédé d'amélioration de la tenue au stockage d'hydroxy- et d'alcoxyalkylcétones liquides selon la revendication 1, caractérisé en ce que l'on utilise, en tant qu'alcoxyalkylcétone, la méthoxy-2-propanone, l'éthoxy-2-propanone, la n-propoxy-2-propanone, l'isopropoxy-2-propanone, la n-butoxy-2-propanone, l'isobutoxy-2-propanone, la sec.-butoxy-2-propanone, la tert.-butoxy-2-propanone, la méthoxy-2-butanone, l'éthoxy-2-butanone, la n-propoxy-2-butanone, l'isopropoxy-2-butanone, la n-butoxy-2-butanone, l'isobutoxy-2-butanone, la sec.-butoxy-2-butanone, la tert.-butoxy-2-butanone ou leurs mélanges.

4. Procédé d'amélioration de la tenue au stockage d'hydroxy- et d'alcoxyalkylcétones liquides selon les revendications 1 à 3, caractérisé en ce qu'on leur ajoute 0,001 à 0,1% en poids d'une base.

5. Procédé d'amélioration de la tenue au stockage d'hydroxy- et d'alcoxyalkylcétones liquides selon les revendications 1 à 4, caractérisé en ce qu'on leur ajoute 0,005 à 0,05% en poids d'une base.

6. Procédé d'amélioration de la tenue au stockage d'hydroxy- et d'alcoxyalkylcétones liquides selon les revendications 1 à 5, caractérisé en ce qu'on utilise, en tant que base, des amines tertiaires, des carbonates alcalins, des hydroxydes alcalins ou leurs mélanges.

7. Procédé d'amélioration de la tenue au stockage d'hydroxy- et d'alcoxyalkylcétones liquides selon les revendications 1 à 6, caractérisé en ce qu'on utilise, en tant que base, la tripropylamine, la tributylamine, la tétraméthylhexane-diamine ou leurs mélanges.

8. Procédé d'amélioration de la tenue au stockage d'hydroxy- et d'alcoxyalkylcétones liquides selon les revendications 1 à 7, caractérisé en ce qu'on utilise, en tant que base, le carbonate de sodium, l'hydroxyde de sodium, le carbonate de potassium, l'hydroxyde de potassium ou leurs mélanges, sous forme solide ou en tant que solution aqueuse.
